Europäisches Patentamt

European Patent Office

Office européen des brevets

(19)

(11) Veröffentlichungsnummer: **0 000 165**
**B1**

(12) **EUROPÄISCHE PATENTSCHRIFT**

(45) Veröffentlichungstag der Patentschrift: 03.06.81

(21) Anmeldenummer: 78100188.8

(22) Anmeldetag: 19.06.78

(51) Int. Cl.³: **C 07 C 39/11,**
C 07 C 37/72, C 07 C 37/20

(54) Verfahren zur Herstellung von reinem 2-Hydroxybenzylalkohol, reinem 4-Hydroxybenzylalkohol oder einem Gemisch beider Hydroxybenzylalkohole.

(30) Priorität: 28.06.77 DE 2729075

(43) Veröffentlichungstag der Anmeldung:
10.01.79 Patentblatt 79/1

(45) Bekanntmachung des Hinweises auf die Patenterteilung:
03.06.81 Patentblatt 81/22

(84) Benannte Vertragsstaaten:
BE CH DE FR NL

(56) Entgegenhaltungen:
NL - A - 254 359
US - A - 3 931 001

(73) Patentinhaber: HAARMANN & REIMER GMBH
Postfach 138
D-3450 Holzminden (DE)

(72) Erfinder: Bauer, Kurt, Dr.
Corveyblick 41
D-3450 Holzminden (DE)
Erfinder: Krempel, Alfred
Dohnenstieg 7
D-3450 Holzminden (DE)
Erfinder: Mölleken, Reiner, Dr.
D-3451 Golmbach-Warbsen, Nr. 16 (DE)
Erfinder: Wedemeyer, Karlfried, Dr.
Bilharzstrasse 7
D-5000 Köln 80 (DE)
Erfinder: Fiege, Helmut, Dr.
Walter-Flex-Strasse 8
D-5090 Leverkusen 1 (DE)

(74) Vertreter: Dill, Erwin, Dr.
c/o BAYER AG Zentralbereich Patente Marken
und Lizenzen Bayerwerk
D-5090 Leverkusen 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 0 000 165

Verfahren zur Herstellung von reinem 2-Hydroxybenzylalkohol, reinem 4-Hydroxybenzylalkohol oder einem Gemisch beider Hydroxybenzylalkohole

Die Erfindung betrifft ein Verfahren zur Herstellung von reinem 2-Hydroxybenzylalkohol, reinem 4-Hydroxybenzylalkohol oder einem Gemisch beider Hydroxybenzylalkohole.

Es ist bekannt, daß 2- und 4-Hydroxybenzylalkohol und Gemische beider Verbindungen durch Umsetzung von Phenol mit Formaldehyd in Gegenwart basischer Katalysatoren erhalten werden. Die Schwierigkeit besteht in der Isolierung der reinen Verbindungen aus den bei der Umsetzung von Phenol mit Formaldehyd anfallenden Reaktionsgemischen.

Bei der Umsetzung von Phenol mit Formaldehyd in Gegenwart basischer Katalysatoren entstehen als Primärprodukte 2- und 4-Hydroxybenzylalkohol; und zwar in Abhängigkeit vom verwendeten Katalysator mehr oder weniger überwiegend 2-Hydroxybenzylalkohol. Infolge ihrer hohen Reaktionsfähigkeit reagieren die Hydroxybenzylalkohole jedoch mit dem im Reaktionsgemisch befindlichen Formaldehyd zu 2,6- und 2,4-Dihydroxymethyl-phenol und 2,4,6-Trihydroxymethyl-phenol, und mit Phenol zu 2,2'-, 2,4'- und 4,4'-Dihydroxy-diphenylmethan. Außerdem kondensieren sie mit sich selber unter Bildung von Dihydroxy-benzyläthern, die ihrerseits, falls die Umsetzung von Phenol mit Formaldehyd bei höheren Temperaturen vorgenommen wird, durch Kondensation in Dihydroxydiphenylmethane übergehen.

Da sich 2- und 4-Hydroxybenzylalkohol nur schwierig von den bei den Sekundärreaktionen entstehenden Nebenprodukten abtrennen lassen, war ihre Reindarstellung aus den bei der Umsetzung von Phenol mit Formaldehyd in Gegenwart basischer Katalysatoren anfallenden Reaktionsgemischen entweder nur mit in technischem Maßstab nicht durchführbaren Verfahren oder unter hohen Ausbeute-Verlusten möglich.

So ist in der britischen Patentschrift 751 845 die Herstellung von 2-Hydroxybenzylalkohol (Saligenin) durch Umsetzung von Phenol mit Formaldehyd in Gegenwart basischer Katalysatoren und Extraktion des anfallenden Umsetzungsproduktes mit Benzol beschrieben. 2-Hydroxybenzylalkohol wird jedoch nur in einer Ausbeute von 50% bezogen auf umgesetztes Phenol erhalten.

In der britischen Patentschrift 774 696 wird die Reindarstellung von 2-Hydroxybenzylalkohol aus Phenol-Formaldehyd-Umsetzungsprodukten durch fraktionierte Kristallisation beschrieben; es wird für das Verfahren eine Ausbeute an Rein-2-Hydroxybenzylalkohol von 21% bezogen auf eingesetzten Formaldehyd angegeben.

Das in der US-Patentschrift 2 804 480 beschriebene Verfahren zur Reindarstellung von 2-Hydroxybenzylalkohol, nämlich die Silylierung des Phenol-Formaldehyd-Umsetzungsproduktes mit Trimethylchlorsilan, fraktionierte Destillation und anschließende Verseifung der Silylierungsprodukte, liefert zwar Hydroxybenzylalkohol von hoher Reinheit, ist aber wegen des technischen Aufwandes und der hohen Kosten unwirtschaftlich und daher lediglich für eine Reindarstellung der Verbindung im Laboratoriumsmaßstab von Interesse.

Es wurde nun überraschenderweise gefunden, daß man 2- und 4-Hydroxybenzylalkohol in hoher Reinheit und ausgezeichneten Ausbeuten auf einfache, wirtschaftliche, auch in technischem Maßstab durchführbare Weise herstellen kann, wenn man Phenol mit Formaldehyd in Gegenwart basischer Katalysatoren umsetzt und das anfallende Reaktionsprodukt, gegebenenfalls nach Entfernen eines Teils des nicht umgesetzten Phenols, einer mindestens 2-stufigen Gegenstromextraktion in einem Lösungsmittelsystem Wasser/mit Wasser nicht oder begrenzt mischbares organisches Lösungsmittel unterwirft, in der entweder in der ersten Extraktionsstufe Phenol und Dihydroxydiphenyl-methane, in der zweiten Extraktionsstufe 2-Hydroxybenzylalkohol oder das Gemisch aus 2- und 4-Hydroxybenzylalkohol und, falls in der zweiten Extraktionsstufe nur 2-Hydroxybenzylalkohol extrahiert wurde, gegebenenfalls in einer dritten Extraktionsstufe 4-Hydroxybenzylalkohol abgetrennt werden oder in der ersten Extraktionsstufe Polyhydroxymethylphenole, in der zweiten Extraktionsstufe 4-Hydroxybenzylalkohol oder das Gemisch aus 2- und 4-Hydroxybenzylalkohol und, falls in der zweiten Extraktionsstufe nur 4-Hydroxybenzylalkohol extrahiert wurde, in einer dritten Extraktionsstufe 2-Hydroxybenzylalkohol abgetrennt werden.

Mit Hilfe der Gegenstromverteilung lassen sich 2- und 4-Hydroxybenzylalkohol in technischem Maßstab in praktisch quantitativer Ausbeute aus den bei der Umsetzung von Phenol mit Formaldehyd anfallenden Reaktionsgemischen isolieren. Da die Ausbeuten an Hydroxybenzylalkoholen bei der Umsetzung von Phenol mit Formaldehyd etwa 70 bis 90% betragen, bezogen auf eingesetzten Formaldehyd, eröffnet das erfindungsgemäße Verfahren einen Weg, reine Hydroxybenzylalkohole insgesamt in einer Ausbeute von 70 bis 85% bezogen auf eingesetzten Formaldehyd in technischem Maßstab herzustellen.

Die Erfindung betrifft daher ein Verfahren zur Herstellung von reinem 2-Hydroxybenzylalkohol und reinen 4-Hydroxybenzylalkohol oder einem Gemisch beider Verbindungen durch Umsetzung von Phenol mit Formaldehyd in Gegenwart eines basischen Katalysators und Extraktion der wäßrigen Lösung des erhaltenen Reaktionsgemisches mit einem organischen Lösungsmittel, gegebenenfalls nach Entfernen eines Teils des nicht umgesetzten Phenols, das dadurch gekennzeichnet ist, daß man das Dihydroxydiphenylmethane, Phenol, 2-Hydroxybenzylalkohol, 4-Hydroxybenzylalkohol, Dihydroxymethylphenole und Polyhydroxymethylphenole enthaltende Reaktionsgemisch einer mindestens 2-

2

# 0 000 165

stufigen Gegenstromextraktion in einem Lösungsmittelsystem Wasser/mit Wasser nicht oder begrenzt mischbares organisches Lösungsmittel unterwirft, wobei man die Gegenstromextraktion in der Weise ausführt, daß man entweder

(a) in der ersten Extraktionsstufe als organische mit Wasser nicht bzw. begrenzt mischbare Lösungsmittel Alkylaromaten mit 7 oder 8 C-Atomen, cyclische Kohlenwasserstoffe mit 6 — 8 C-Atomen oder aliphatische 6 — 8 C-Atome aufweisende Äther einsetzt und ein Volumenverhältnis $V_{\text{Lösungsmittel}}/V_{\text{Wasser}}$ anwendet, so daß sich eine Phenol und Dihydroxydiphenylmethane enthaltende organische und eine die übrigen Verbindungen enthaltende wäßrige Phase bildet, die organische Phase abtrennt und die wäßrige Phase in der zweiten Extraktionsstufe einer Gegenstromverteilung mit aliphatischen 6 — 8 C-Atome aufweisenden Äthern oder aliphatischen 5 — 6 C-Atome aufweisenden Ketonen unterwirft, so daß sich eine organische Phase, die entweder 2-Hydroxybenzylalkohol oder 2- und 4-Hydroxybenzylalkohol enthält und eine wäßrige Phase, die die restlichen Verbindungen enthält, bildet, die organische Phase abtrennt und, falls in der zweiten Extraktionsstufe 2-Hydroxybenzylalkohol abgetrennt wurde, die wäßrige Phase in einer dritten Extraktionsstufe einer Gegenstromverteilung mit aliphatischen, 5 — 6 C-Atome aufweisenden Ketonen oder aliphatischen 4 — 8 C-Atome aufweisenden Alkoholen unterwirft, so daß sich eine den 4-Hydroxybenzylalkohol enthaltende organische Phase und eine die Polyhydroxymethylphenole enthaltende wäßrige Phase bildet, oder

(b) in der ersten Extraktionsstufe als organische mit Wasser nicht bzw. begrenzt mischbare Lösungsmittel aliphatische 5 — 6 C-Atome aufweisende Ketone einsetzt und ein Volumenverhältnis $V_L/V_W$ anwendet, so daß sich eine die Polyhydroxymethylphenole enthaltende wäßrige Phase und eine die übrigen Verbindungen enthaltende organische Phase bildet, die wäßrige Phase abtrennt und die organische Phase in einer zweiten Extraktionsstufe einer Gegenstromverteilung mit Wasser unterwirft, so daß sich eine wäßrige Phase, die entweder 4-Hydroxybenzylalkohol oder 4- und 2-Hydroxybenzylalkohol enthält, und eine organische Phase bildet, die die übrigen Verbindungen enthält, die wäßrige Phase abtrennt und, falls in der zweiten Extraktionsstufe nur 4-Hydroxybenzylalkohol extrahiert wurde, die organische Phase in einer dritten Extraktionsstufe einer Gegenstromverteilung mit Wasser unterwirft, so daß sich eine den 2-Hydroxybenzylalkohol enthaltende organische und eine die restlichen Verbindungen enthaltende wäßrige Phase bildet.

Die Umsetzung von Phenol und Formaldehyd in Gegenwart der basischen Katalysatoren kann grundsätzlich unter den aus der Literatur bekannten Reaktionsbedingungen erfolgen. Es hat sich aber für das erfindungsgemäße Verfahren als vorteilhaft erwiesen, bestimmte Reaktionsbedingungen einzuhalten.

So ist es von Vorteil, abweichend von den üblichen Reaktionsbedingungen, mit einem hohen Phenolüßerschuß, z.B. mit einem Molverhältnis Phenol:Formaldehyd von 5—15:1, vorzugsweise 8—12:1 zu arbeiten. Auf diese Weise kann die Bildung von Dihydroxymethylphenolen und Dihydroxydiphenylmethanen stark verringert werden.

Ferner hat sich die Verwendung von Paraformaldehyd als vorteilhaft erwiesen. Durch ihn kann der Wassergehalt der Reaktionsmischung unter 5% gehalten werden. Dadurch wird eine Steigerung der Umsetzung und eine Erhöhung der Reaktionsgeschwindigkeiten erreicht und die Abtrennung des überschüssigen Phenols erleichtert.

Als basische Katalysatoren kommen Alkali- und Erdalkalihydroxide, wie Natriumhydroxid, Kaliumhydroxid, Lithiumhydroxid, Calciumhydroxid oder Bariumhydroxid, Alkaliphenolate, ferner Oxide und Salze organischer Säuren wie Essigsäure, Ameisensäure und Milchsäure, zweiwertiger Metalle wie Magnesiumoxid, Zinkoxid, Bleioxid, Zinkacetat, Magnesiumacetat usw. in Frage. Ebenfalls verwendet werden können basische Stickstoffverbindungen, z.B. Ammoniumsalze und Amine wie Triäthanolamin und Hexamethylentetramin. Die Auswahl des Katalysators richtet sich im wesentlichen nach der gewünschten Zusammensetzung des Reaktionsproduktes. So wird bei der Verwendung von Salzen zweiwertiger Metalle fast nur 2-Hydroxybenzylalkohol neben geringen Mengen 4-Hydroxybenzylalkohol gebildet, während starke Basen wie Natrium- und Kaliumhydroxid den Anteil an 4-Hydroxybenzylalkohol bis auf etwa 30 Gew.-% und mehr, bezogen auf die Gesamtmenge der im Reaktionsprodukt enthaltenen Hydroxybenzylalkohole, ansteigen lassen.

Es hat sich bewährt, die Katalysatoren in Mengen von 0,05 bis 0,0001 Mol, vorzugsweise in Mengen von 0,01 bis 0,0005 Mol, je Mol Phenol einzusetzen.

Die Reaktionstemperatur kann zwischen 25 und 120°C schwanken. Im allgemeinen hat es sich in dem erfindungsgemäßen Verfahren als vorteilhaft erwiesen, die Umsetzung von Phenol mit Formaldehyd bei Temperaturen von 40 bis 90°C durchzuführen.

Bei Einhaltung dieser vorstehend als vorteilhaft hervorgehobenen Reaktionsbedingungen werden Umsätze bis nahezu 100%, bezogen auf eingesetzten Formaldehyd, erhalten und dies bei Reaktionszeiten von 0,5 bis 5 Stunden.

Die Umsetzung von Phenol mit Formaldehyd kann kontinuierlich und diskontinuierlich durchgeführt werden.

Das bei der Umsetzung von Phenol mit Formaldehyd anfallende Reaktionsgemisch kann, insbesondere wenn die Umsetzung ohne größeren Phenolüberschuß durchgeführt wurde, ohne weitere Vorbehandlung der Gegenstromextraktion unterworfen werden. Wurde für die Umsetzung dagegen ein

3

# 0 000 165

größerer Phenolüberschuß, z.B. 5 bis 15 Mol Phenol je Mol Paraformaldehyd angewendet, hat es sich als vorteilhaft erwiesen, vor der Gegenstromextraktion aus dem Reaktionsgemisch soviel Phenol abzutrennen, daß die Phenolmenge im zu extrahierenden Produkt nur noch 50 Gew.-% oder weniger beträgt. Die Abtrennung des Phenols kann durch Destillation und/oder Ausfrieren erfolgen. Es hat sich als vorteilhaft erwiesen, erst das nach dem Ausfrieren der Hauptmenge Phenol zurückbleibende Reaktionsgemisch einer kontinuierlichen Vakuumdestillation, z.B. in einem Fallfilmverdampfer zu unterwerfen. Dabei werden außerdem nicht umgesetzter Formaldehyd, Wasser und weiteres Phenol zurückgewonnen. Das zurückgewonnene Phenol kann erneut zur Umsetzung mit Formaldehyd verwendet werden.

Es hat sich bewährt, vor dem Abtrennen des Phenols, den zugesetzten Katalysator zu desaktivieren bzw. zu entfernen. Dies kann durch vorsichtige Neutralisation, z.B. mit organischen Säuren wie Essigsäure, oder Ionenaustauschern geschehen.

Die Abtrennung der Hydroxybenzylalkohole aus dem verbleibenden Reaktionsgemisch erfolgt durch Gegenstromextraktion in einem System Wasser/mit Wasser nicht oder begrenzt mischbares organisches Lösungsmittel. Dabei werden das Lösungsmittel und das Volumenverhältnis $V_{Lösungsmittel}$, im folgenden als $V_L$ bezeichnet, : $V_{Wasser}$, im folgenden als $V_W$ bezeichnet, so gewählt, daß entweder die in der jeweiligen Extraktionsstufe abzutrennende Verbindung (bzw. Verbindungen) praktisch vollständig in die organische Phase übergeht und die übrigen Reaktionsprodukte in der wäßrigen Phase verbleiben oder die abzutrennende Verbindung (bzw. Verbindungen) in die wäßrige Phase übergeht und die übrigen Reaktionsprodukte in der organischen Phase verbleiben.

Es hat sich als vorteilhaft erwiesen, insbesondere bei Reaktionsgemischen, die größere Mengen Phenol enthalten, in der ersten Stufe der Gegenstromextraktion Phenol und Dihydroxydiphenylmethane abzutrennen. Als geeignete organische Lösungsmittel haben sich hierbei für das System Wasser/mit Wasser nicht oder begrenzt mischbares organisches Lösungsmittel, vor allem Alkylaromaten mit 7 oder 8 C-Atomen, wie Toluol, o-, m- und p-Xylol und Äthylbenzol, cyclische Kohlenwasserstoffe mit 6 bis 8 C-Atomen, wie Cyclohexan, Cycloheptan und Cyclooctan, aliphatische, 6 bis 8 C-Atome aufweisende Äther, wie Di-n-propyläther, Di-n-butyläther und Di-isopropyläther, sowie Gemische dieser Lösungsmittel erwiesen. Besonders bewährt haben sich die Alkylaromaten mit 7 bis 8 C-Atomen. Die übrigen Reaktionsprodukte des Reaktionsgemisches verbleiben in der wäßrigen Phase.

Soll in der zweiten Extraktionsstufe nur 2-Hydroxybenzylalkohol abgetrennt werden, so extrahiert man vorteilhaft die in der ersten Extraktionsstufe angefallene wäßrige Phase mit den vorstehend genannten, 6 bis 8 C-Atome aufweisenden aliphatischen Äthern — allerdings unter Anwendung eines größeren Volumenverhältnisses $V_L/V_W$ als in der ersten Extraktionsstufe — oder mit aliphatischen, 5 bis 6 C-Atome aufweisenden Ketonen, wie Diäthylketon, Äthylpropylketon, Äthylisopropylketon, Methylbutylketon und Methyl-iso-butylketon. Vorzugsweise werden die aliphatischen 6 bis 8 C-Atome aufweisenden Äther verwendet.

Der nach der Extraktion des 2-Hydroxybenzylalkohols in der wäßrigen Phase neben den Polyhydroxy-methylphenolen verbleibende 4-Hydroxybenzylalkohol kann gegebenenfalls in einer dritten Extraktionsstufe, z.B. mit den vorstehend genannten, 5 bis 6 C-Atome aufweisenden aliphatischen Ketonen — allerdings unter Anwendung eines größeren Volumenverhältnisses $V_L/V_W$ als in der zweiten Extraktionsstufe — oder mit aliphatischen, 4 bis 8 C-Atome aufweisenden Alkoholen wie Butanol-(1), Butanol-(2), Pentanol-(1), Pentanol-(2), 3-Methyl-butanol-(1), Hexanol-(1), Heptanol-(1), Heptanol-(2) extrahiert werden. Besonders bevorzugt sind hierbei die aliphatischen, 5 bis 6 C-Atome aufweisenden Ketone.

Sollen dagegen in der zweiten Extraktionsstufe gleichzeitig 2- und 4-Hydroxybenzylalkohol abgetrennt werden, so extrahiert man vorteilhaft die in der ersten Extraktionsstufe angefallene wäßrige Phase mit den vorstehend genannten aliphatischen, 5 bis 6 C-Atome aufweisenden Ketonen — allderdings unter Anwendung eines größeren Volumenverhältnisses $V_L/V_W$ als bei der Extraktion des 2-Hydroxybenzylalkohols — oder 4 bis 8 C-Atome aufweisenden Alkoholen. Vorzugsweise werden die 5 bis 6 C-Atome aufweisenden aliphatischen Ketone verwendet. Die organische Phase enthält dann das Gemisch aus 2- und 4-Hydroxybenzylalkohol, während die polareren Polyhydroxymethyl-phenole in der wäßrigen Phase verbleiben, aus der sie gewünschtenfalls in einer nachgeschalteten Extraktion isoliert werden können.

Enthält das aufzutrennende Reaktionsgemisch z.B. wegen der bei der Reaktion angewendeten Molverhältnisse kein oder nur wenig Phenol, kann es vorteilhaft sein, die Reaktionskomponenten in umgekehrter Reihenfolge zu isolieren, d.h. in der ersten Extraktionsstufe Polyhydroxymethyl-phenole, in der zweiten Extraktionsstufe 4-Hydroxybenzylalkohol oder das Gemisch aus 2- und 4-Hydroxybenzylalkohol und, falls in der zweiten Stufe nur 4-Hydroxybenzylalkohol extrahiert wurde, in der dritten Extraktionsstufe 2-Hydroxybenzylalkohol abzutrennen. Da bei dieser Arbeitsweise die abzutrennenden Verbindungen stets in der wäßrigen Phase anfallen, ist es zweckmäßig, für diese Gegenstromextraktion organische Lösungsmittel solcher Polarität auszuwählen, daß bereits eine Änderung des Volumenverhältnisses $V_L/V_W$ ausreicht, um das Verteilungsgleichgewicht für die einzelnen Verbindungen in einer für ihre Trennung ausreichenden Weise zu verändern. Solche Lösungsmittel können dann in allen zwei bzw. drei Extraktionsstufen eingesetzt werden. Es ist in den einzelnen Extraktionsstufen lediglich eine Änderung des Volumenverhältnisses $V_L/V_W$ erforderlich und es wird

4

vermieden, daß die in der organischen Phase verbleibenden Reaktionskomponenten für jede Extraktionsstufe in ein anderes organisches Lösungsmittel überführt werden müssen.

Organische Lösungsmittel dieser Polarität sind z.B. die vorstehend genannten, aliphatischen, 5 bis 6 C-Atome aufweisenden Ketone, insbesondere Methyl-isobutylketon.

Bei der letztgenannten Arbeitsweise wird das aufzutrennende Reaktionsgemisch in der ersten Extraktionsstufe unter Anwendung eines relativ großen Volumenverhältnis $V_L/V_W$ extrahiert; dabei gehen die Polyhydroxymethylphenole in die wäßrige Phase über, während die Hydroxybenzylalkohole zusammen mit den Dihydroxydiphenylmethanen und gegegebenenfalls vorhandenem Phenol in der organischen Phase verbleiben.

Aus der organischen Phase werden dann entweder unter Anwendung abgestuft kleiner werdender Volumenverhältnisse $V_L/V_W$ in einer zweiten und dritten Extraktionsstufe 4-Hydroxybenzylalkohol und 2-Hydroxybenzylalkohol getrennt mit Wasser extrahiert oder es wird unmittelbar mit dem kleineren Volumenverhältnis das Gemisch aus 4- und 2-Hydroxybenzylalkohol mit Wasser extrahiert; Dihydroxydiphenylmethane und gegebenenfalls vorhandenes Phenol verbleiben in der organischen Phase.

Es ist für den Fachmann selbstverständlich, daß für die einzelnen Extraktionsstufen über die beispielsmäßig genannten organischen Lösungsmittel bzw. Lösungsmittelgemische hinaus auch andere organische Lösungsmittel eingesetzt werden können, falls diese neben einer ausreichenden Selektivität auch eine hinreichende Kapazität für die zu trennenden Verbindungen aufweisen.

Die erfindungsgemäße Gegenstromextraktion kann diskontinuierlich oder kontinuierlich durchgeführt werden. Im allgemeinen hat es sich bewährt, wenn in dem System Wasser/mit Wasser nicht oder begrenzt mischbares organisches Lösungsmittel das Volumenverhältnis $V_L/V_W$ etwa 0,05 bis 20 beträgt. Die kontinuierliche Gegenstromextraktion erfordert im allgemeinen aus technischen Gründen die Einhaltung von Volumenverhältnissen von etwa 0,05 bis 20.

Werden bei der Umsetzung von Phenol mit Formaldehyd Reaktionsgemische erhalten, die im wesentlichen 2-Hydroxybenzylalkohol und nur geringe Mengen 4-Hydroxybenzylalkohol enthalten, kann es aus wirtschaftlichen Gründen zweckmäßig sein, den 4-Hydroxybenzylalkohol nicht wie beschrieben in einer gesonderten Extraktionsstufe abzutrennen, sondern zusammen mit den Polyhydroxymethylphenolen zu verwerfen.

Die in der organischen Phase anfallenden 2- und 4-Hydroxybenzylalkohole bzw. das Gemisch beider Verbindungen werden durch Abdestillieren des Lösungsmittels isoliert. Fallen sie dagegen in der wäßrigen Phase an, so müssen sie zunächst aus dieser mit einem organischen Lösungsmittel extrahiert und dann aus diesem wie beschrieben isoliert werden.

Die erfindungsgemäß anzuwendende Gegenstromextraktion kann in an sich bekannten Extraktionsvorrichtungen durchgeführt werden, z.B. in Kolonnen ohne Rührer, wie Extraktions-Sprühtürmen oder Extraktions-Türmen mit oder ohne Füllkörper, oder in Kolonnen mit Rührern wie Scheibel-Kolonnen oder RDC-Kolonnen (Rotating Disc Contactor) oder in Extraktionszentrifugen. Für die kontinuierliche Gegenstromextraktion haben sich Siebboden-Pulsationskolonnen oder RDC-Kolonnen als besonders vorteilhaft erwiesen.

Die Extraktions-Temperatur für die einzelnen Extraktionsstufen kann in weiten Grenzen schwanken. Aus praktischen Gründen arbeitet man im allgemeinen bei Temperaturen von 20 bis 70°C, vorzugsweise von 50 bis 60°C. Die Extraktionen werden aus praktischen und wirtschaftlichen Gründen im allgemeinen bei Normaldruck bzw. bei Verwendung von besonders niedrig siedenden Extraktionsmitteln unter Überdruck durchgeführt.

Die nach dem erfindungsgemäßen Verfahren hergestellten Verbindungen sind wichtige Zwischenprodukte für die Herstellung organischer Verbindungen. So ist 2-Hydroxybenzylalkohol ein wichtiges Ausgangsmaterial für die Herstellung von Salicylaldehyd, 4-Hydroxybenzylalkohol ein wichtiges Ausgangsmaterial für die Herstellung von 4-Hydroxybenzaldehyd, Anisalkohol und Anisaldehyd.

Bei den in den folgenden Beispielen und Tabellen engegebenen Prozenten handelt es sich, sofern nichts anderes angegeben ist, um Gewichts-%.

Beispiel 1
Herstellung der zu extrahierenden Phenol-Formaldehyd-Reaktionsgemische
(Roh-Hydroxybenzylalkohole)

(a) 10,7 Mol Phenol wurden mit 1 Mol Paraformaldehyd (Wassergehalt: 3,6%) und Rühren auf 50°C erwärmt. Nach Zugabe von 0,039 g Lithiumhydroxid je Mol Phenol wurde die Reaktionsmischung 5 Stunden auf 50°C gehalten. Nach Neutralisation des Lithiumhydroxids mit der äquivalenten Menge Essigsäure wurde ein Teil des überschüssigen Phenols zunächst im Fallfilmverdampfer, danach im Dünnschichtverdampfer bei 55 bis 60°C und 3—5 Torr abdestilliert.

Das auf diese Weise erhaltene Reaktionsgemisch wies folgende Zusammensetzung auf:

2,81 % Dihydroxydiphenylmethane

50,64 % Phenol

# 0 000 165

38,80 % 2-Hydroxybenzylalkohol

4,33 % 4-Hydroxybenzylalkohol

2,94 % 2,6-Dihydroxymethylphenol

<0,01 % 2,4-Dihydroxymethylphenol und Polyhydroxymethylphenole

0,07 % Wasser

0,41 % Lithiumacetat

Die Zusammensetzung des erhaltenen Reaktionsgemisches wurde durch Hochdruckflüssigkeitschromatographie bzw. — nach Überführung der Komponenten des Reaktionsgemisches in ihre Trimethylsilylderivate mittels N-Methyl-N-trimethylsilyl-acetamid — durch Gaschromatographie bestimmt.

Der Umsatz, bezogen auf eingesetzten Paraformaldehyd, betrug etwa 98%. Der Formaldehyd-Umsatz wurde polarographisch bestimmt.

Das Reaktionsgemisch wurde in Form 10- und 20%iger Lösungen in destilliertem Wasser der Gegenstromextraktion unterworfen.

(b) Es wurde wie unter a) beschrieben verfahren, mit dem Unterschied, daß anstelle von 10,7 Mol Phenol nur 9 Mol Phenol eingesetzt wurden, daß als Katalysator Zinkacetat (0,208 g Zinkacetat je Mol Phenol) verwendet und die Reaktion 8 Stunden bei 65°C ausgeführt wurde.

Das auf diese Weise erhaltene Reaktionsgemisch wies folgende Zusammensetzung auf:

2,92 % Dihydroxydiphenylmethane

49,97 % Phenol

42,64 % 2-Hydroxybenzylalkohol

0,89 % 4-Hydroxybenzylalkohol

3,08 % 2,6-Dihydroxymethylphenol

<0,01 % 2,4-Dihydroxymethylphenol und Polyhydroxymethylphenole

0,07 % Wasser

0,44 % Zinkacetat

Das Reaktionsgemisch wurde in Form einer 15%igen Lösung in destilliertem Wasser der Gegenstromextraktion unterworfen.

(c) Am Boden eines wärmeisolierten, mit Raschigringen gefüllten Reaktionsturmes wurden je Stunde eine auf etwa 60 bis 65°C vorgewärmte Lösung von 25 Mol Paraformaldehyd (Wassergehalt: 3,6%) in 250 Mol Phenol und eine Lösung von 0,6 Mol Kaliumhydroxid in 10 Mol Phenol unter Vermischen gleichmäßig eingepumpt. Das nach einer mittleren Verweilzeit von einer Stunde aus dem Reaktionsturm kontinuierlich austretende Reaktionsgemisch wies, nachdem sich ein stationärer Zustand eingestellt hatte, eine Temperatur von etwa 75°C auf. Der Umsatz, bezogen auf Formaldehyd, betrug etwa 98%.

Durch Abkühlen des Reaktionsgemisches auf 20 bis 25°C wurde etwa die Hälfte des eingesetzten Phenols ausgefroren. Das ausgefrorene Phenol wurde durch Zentrifugieren von der Mutterlauge getrennt und in die Reaktion zurückgeführt.

Das in der Mutterlauge enthaltene Kaliumhydroxid wurde mit Essigsäure neutralisiert.

Die vereinigten neutralisierten Mutterlaugen, die in der vorstehend beschriebenen Weise innerhalb einer Betriebszeit von 22 Stunden angefallen waren, wurden durch Destillation im Dünnschichtverdampfer bei 55 bis 60°C/3—5 Torr von weiterem Phenol befreit. Das nach der Destillation zurückbleibende Sumpfprodukt (132,7 kg) wies folgende Zusammensetzung auf:

2,88 % Dihydroxydiphenylmethane

49,51 % Phenol

30,53 % 2-Hydroxybenzylalkohol

6

13,09 % 4-Hydroxybenzylalkohol

2,13 % 2,6-Dihydroxymethylphenol

0,87 % 2,4-Dihydroxymethylphenol und Polyhydroxymethylphenole

0,05 % Wasser

0,94 % Kaliumacetat

Das Sumpfprodukt wurde als solches und in Form 20- bzw. 25%iger Lösungen in destilliertem Wasser der Gegenstromverteilung unterworfen.

(d) Die Mischung aus 3 Mol Phenol, 1 Mol Paraformaldehyd (Wassergehalt: 3,6%) und 0,12 g Kaliumhydroxid je Mol Phenol wurde unter Rühren auf 70°C erwärmt und 6 Stunden auf dieser Temperatur gehalten. Der Umsatz bezogen auf Paraformaldehyd, betrug etwa 99%.

Das Reaktionsgemisch wurde wie in Beispiel 1 (a) beschrieben aufgearbeitet und analysiert. Es wies folgende Zusammensetzung auf:

6,07 % Dihydroxydiphenylmethane

17,02 % Phenol

41,90 % 2-Hydroxybenzylalkohol

17,95 % 4-Hydroxybenzylalkohol

11,78 % 2,6-Dihydroxymethylphenol

4,80 % 2,4-Dihydroxymethylphenol und Polyhydroxymethylphenole

0,01 % Wasser

0,47 % Kaliumacetat

Das Reaktionsgemisch wurde in Form einer 20%igen Lösung in destilliertem Wasser der Gegenstromextraktion unterworfen.

Beispiel 2
Erste Extraktionsstufe der Gegenstromextraktion

1 l der 25%igen wäßrigen Lösung des in Beispiel 1 c) erhaltenen Sumpfproduktes wurde in einer 14 Stufen umfassenden Extraktionsapparatur einer Craig-Verteilung (s. Houben-Weyl, Methoden der organischen Chemie, Band I/1, 1958, Seite 257 und ff.) unterworfen.

Dazu wurde die zu extrahierende Lösung in Element 1 der Apparatur vorgelegt und bei einer Temperatur von 60°C mit Toluol unter Anwendung eines Volumenverhältnisses $V_L/V_w$ von 1,05/1 extrahiert.

Die Analyse der wäßrigen (Raffinat-)Phase und der Lösungsmittel-(Solvent-) Phase ergab folgende Verteilung der Komponenten:

Elemente 1—7:
Vereinigte Solventphasen:

100,8 g (= 99,8%) Phenol und Dihydroxydiphenylmethane

0,2 g (= 0,2%) 2-Hydroxybenzylalkohol

Elemente 8—14:
Vereinigte Raffinatphasen:

0,35 g (= 0,36%) Phenol und Dihydroxydiphenylmethane

58,76 g (= 59,88%) 2-Hydroxybenzylalkohol

39,02 g (= 39,76%) 4-Hydroxybenzylalkohol und Di- und Polyhydroxymethylphenole.

7

## 0 000 165

Der Inhalt der nicht aufgeführten Elemente wurde verworfen.

Aus den Daten geht hervor, daß in der ersten Extraktionsstufe des erfindungsgemäßen Verfahrens die Abtrennung von Phenol und Dihydroxydiphenylmethanen von den Hydroxybenzylalkoholen und den Di- und Polyhydroxymethylphenolen erreicht wird.

Die Abtrennung der Hydroxybenzylalkohole aus den vereinigten Raffinatphasen in der zweiten Extraktionsstufe wird in den nachfolgenden Beispielen 12, 13, 14, 16, 25 und 30 beschrieben.

### Beispiele 3—10
### Erste Extraktionsstufe der Gegenstromextraktion

Wäßrige Lösungen mit Feststoffgehalten zwischen 10 und 25% der nach den Beispielen 1 a) bis d) erhaltenen Reaktionsgemische wurden unter Verwendung verschiedener organischer Lösungsmittel bei Temperaturen zwischen 25 und 60°C in Extraktionsapparaturen nach Craig extrahiert.

In der nachstehenden Tabelle I sind in Spalte 1 die Menge an eingesetzter wäßriger Lösung, der Feststoffgehalt sowie die Zusammensetzung des Feststoffes entsprechend den Beispielen 1 a) bis d), in Spalte 2 die für die Extraktion angewandten Lösungsmittel und Lösungsmittelmengen sowie die Temperatur, in Spalte 3 die Anzahl der Extraktionsstufen, in Spalte 4 und 5 der Gehalt der Solvent- bzw. Raffinat-Phasen an Phenol und Dihydroxydiphenylmethanen (A), 2-Hydroxybenzylalkohol (B) und 4-Hydroxybenzylalkohol und Di- und Polyhydroxymethylphenolen (C) sowie die zur Bestimmung des Gehaltes verwendeten Fraktionen (gewonnene Fraktionen) aufgeführt. Die nicht aufgeführten Fraktionen wurden verworfen. Die Lösungsmittelmengen beziehen sich auf jeweils eine Extraktionsstufe.

Aus den Daten geht hervor, daß in der ersten Extraktionsstufe des erfindungsgemäßen Verfahrens eine Abtrennung von Phenol und Dihydroxydiphenylmethanen von den Hydroxybenzylalkoholen und den Di- und Polyhydroxymethylphenolen erreicht wird.

Die Abtrennung der Hydroxybenzylalkohole aus den vereinigten Raffinatphasen ist in den nachfolgenden Beispielen 11, 15, 17—23, 26—29 beschrieben.

TABELLE I

| Beispiel | Menge der wässr. Lösung [Liter] Feststoffgehalt [%] aus Beispiel 1 | org. Lösungsmittel/ Menge [Liter]/ Temperatur [°C] 2 | Extraktions stufen 3 | Solventphase | | Raffinatphase | |
|---|---|---|---|---|---|---|---|
| | | | | gewonnene Fraktionen | Gehalt 4 | gewonnene Fraktionen | Gehalt 5 |
| 3 | 0,05/20/1a | Cyclohexan/ 0,6/60°C | 24 | 13 — 24 | A= 3,79g(=99,62%) B= 0,01g(= 0.38%) C= 0,00g(= 0.00%) | 1 — 12 | A= 0,02 g(= 0,58%) B= 2,75 g(=80,90%) C= 0,63 g(=18,52%) |
| 4 | 0,1 /15/1b | Benzol/ 0,15/25°C | 14 | 8 — 14 | A= 6.06g(=99.68%) B= 0,02g(= 0,32%) C= 0,00g(= 0,00%) | 1 — 7 | A= 0,02 g(= 0,44%) B= 4:89 g(=88,83%) C= 0,59 g(=10,73%) |
| 5 | 1 /10/1a | Benzol/ 1,11/60°C | 15 | 8 — 15 | A=39,72g(=99,09%) B= 0,36g(= 0,91%) C= 0,00g(= 0,00%) | 1 — 8 | A= 0,50 g(= 1,38%) B=28,83 g(=79.27%) C= 7,03 g(=19,35%) |
| 6 | 0,05/20/1d | o-Xylol/ 0,02/60°C | 31 | 17 — 31 | A= 1,56g(=99,30%) B= 0,11g(= 0,70%) C= 0,00g(= 0.00%) | 1 — 15 | A= 0,006g(= 0,1 %) B= 2,84 g(=46,48%) C= 3,26 g(=53,42%) |
| 7 | 0,1 /20/1c | p-Xylol/ 0,18/60°C | 26 | 14:— 26 | A= 7,12g(=99,46%) B= 0,39g(= 0,54%) C= 0,00g(= 0,00%) | 1 — 13 | A= 0,06 g(= 0,93%) B= 4,15 g(=58.77%) C= 2,85 g(=40,30%) |
| 8 | 1 /25/1c | Aethylbenzol/ 1,61/60°C | 15 | 8 — 15 | A=98,85g(=99,44%) B= 0,56g(= 0,56%) C= 0,00g(= 0,00%) | 1 — 8 | A= 0,96 g(= 0,99%) B=57,59 g(=59,57%) C=38,12 g(=39,44%) |
| 9 | 0,1 /10/1a | Diisopropyläther/ 0,01/25°C | 10 | 6 — 10 | A= 4;29g(=99,76%) B= 0,01g(= 0,24%) C= 0,00g(= 0,00%) | 1 — 5 | A= 0,01 g(= 0,37%) B= 3,11 g(=81,33%) C= 0,70 g(=18,30%) |
| 10 | 0,1 /15/1b | Diisopropyläther/ 0,016/55°C | 13 | 7 — 13 | A= 6,18g(=99,37%) B= 0,04g(= 0,63%) C= 0,00g(= 0,00%) | 1 — 7 | A= 0,05 g(= 0,85%) B= 4,98 g(=88.98%) C= 0,57 g(=10,16%) |

0 000 165

## 0 000 165

Beispiele 11—30
Zweite Extraktionsstufe der Gegenstromextraktion

Die in den Beispielen 2, 5 und 8 erhaltenen, nahezu Phenol und Dihydroxydiphenylmethan freien wäßrigen Phasen werden in der zweiten Extraktionsstufe mit verschiedenen Lösungsmitteln extrahiert.

Analog Tabelle I sind in der nachstehenden Tabelle II, Spalte 1, die Menge an eingesetzter wäßriger Lösung, der Feststoffgehalt sowie die Zusammensetzung des Feststoffs entsprechend den Beispielen 2, 5 und 8, in Spalte 2, das für die Extraktion angewendete Lösungsmittel, die Lösungsmittelmenge und die Temperatur, in Spalte 3 die Anzahl der Extraktionsstufen und in Spalten 4 und 5 die zur Bestimmung des Gehaltes ausgewählten Fraktionen und ihr Gehalt aufgeführt. Die Bezeichnungen A, B und C haben die in den Erläuterungen für Tabelle I angegebene Bedeutung. Die angegebenen Lösungsmittelmengen beziehen sich jeweils auf eine Extraktionsstufe.

Aus den angegebenen Daten geht hervor, daß in der zweiten Extraktionsstufe der Gegenstromextraktion eine Abtrennung des 2-Hydroxybenzylalkohols von 4-Hydroxybenzylalkohol und Di- und Polyhydroxymethylphenolen erreicht wird. Mit Hilfe des erfindungsgemäßen Verfahrens wird 2-Hydroxybenzylalkohol in hoher Reinheit und praktisch verlustfrei aus den Reaktionsgemischen erhalten.

TABELLE II

| Beispiel | Menge der wässr. Lösung [Liter] Fest-stoffgehalt [%] aus Beispiel 1 | org. Lösungsmittel/ Menge [Liter]/ Temperatur [°C] 2 | Extrak-tions stufen 3 | Solventphase | | Raffinatphase | |
|---|---|---|---|---|---|---|---|
| | | | | gewonnene Fraktionen | Gehalt 4 | gewonnene Fraktionen | Gehalt 5 |
| 11 | 1 /0,46/5 | Diisopropyläther 0,99/25° | 30 | 16 – 30 | A=0,06 g(= 2,41%) B=2,49 g(=97,47%) C=0,003g(= 0,12%) | 1 – 15 | A= 0,00g(= 0,00%) B= 0,01g(= 1,93%) C= 0,63g(=98,07%) |
| 12 | 1 /1,4 /2 | Diisopropyläther/ 1,35/55° | 31 | 17 – 31 | A=0.05 g(=0.84%) B=5,69 g(=98,91%) C=0,01 g(= 0,25%) | 1 – 15 | A= 0,00g(= 0.00%) B= 0,02g(= 0,57%) C= 3,77g(=99,43%) |
| 13 | 0,5/1,4/2 | Di-n-propyläther/ 1,04/55° | 28 | 15 – 28 | A=0,02 g(= 0,82%) B=2,90 g(=98,81%) C=0,01 g(= 0,38%) | 1 – 14 | A= 0,00g(= 0,00%) B= 0,02g(= 0,86%) C= 1,93g(=99.14%) |
| 14 | 0,5/1,4/2 | Di-n-butyläther/ 1.59/55° | 27 | 15 – 27 | A=0,02 g(= 0,79%) B=2,93 g(=99,03%) C=0,005g(= 0.17%) | 1 – 13 | A= 0,00g(= 0,00%) B= 0,008g(= 0.39%) C= 1,95g(=99.61%) |
| 15 | 0,5/1,38/8 | Pentanon-(2)/ 0,065/55° | 86 | 45 – 70 | A=0,003g(= 0,12%) B=2,41 g(=99,30%) C=0,014g(= 0,58%) | 1 – 42 | A= 0.00g(= 0.00%) B= 0.02g(= 1,27%) C= 1,60g(=98,73%) |
| 16 | 1 /1,4/2 | 4-Methyl-pentanon-(2)/ 0,15/25° | 54 | 29 – 54 | A=0,05 g(= 0,87%) B=5,24 g(=98,84%) C=0,01 g(= 0,28%) | 1 – 26 | A= 0,00g(= 0.00%) B= 0,02g(= 0,65%) C= 3,48g(=99,35%) |
| 17 | 0,5/0,46/5 | 4-Methyl-pentanon-(2)/ 0,1/55° | 80 | 41 – 70 | A=0,001g(= 0,1 %) B=1,03 g(=99.9 %) C=0,00 g(= 0.00%) | 1 – 30 | A= 0,00g(= 0.00%) B= 0,00g(= 0.01%) C= 0,31g(=99,99%) |

0 000 165

TABELLE II (Fortsetzung)

| Beispiel | Menge der wässr. Lösung [Liter] Fest-stoffgehalt [%] aus Beispiel | org. Lösungsmittel/ Menge [Liter]/ Temperatur [°C] | Extraktions stufen | Solventphase | | Raffinatphase | |
|---|---|---|---|---|---|---|---|
| | | | | gewonnene Fraktionen | Gehalt | gewonnene Fraktionen | Gehalt |
| | 1 | 2 | 3 | 4 | | 5 | |
| 18 | 0,5/1,38/8 | 2-Methyl-pentanon-(3)/ 0,13/55° | 68 | 36 — 59 | A=0,001 g(= 0,04%) B=2,48 g(=99.52%) C=0,01 g(= 0,44%) | 1 — 33 | A= 0.00g (= 0.00%) B= 0,01g (= 0,96%) C= 1.64g (=99.04%) |
| 19 | 0,5/0,46/3 | 1,7-Dimethyl-heptanon-(4)/ 0,36/55° | 48 | 26 — 42 | A=0.002g(= 0,17%) B=1,16 g(=99,74%) C=0,001 g(= 0,09%) | 1 — 23 | A=0,00 g(= 0,00%) B=0,003g(= 1,03%) C=0,28 g(=98,97%) |
| 20 | 0,5/1,38/8 | Essigsäureäthyl= ester/ 0,11/55° | 86 | 44 — 70 | A=0,00 g(= 0.00%) B=2,33 g(=98,02%) C=0,05 g(= 1,98%) | 1 — 43 | A=0,00 g(= 0.00%) B=0,07 g(= 4,41%) C=1,54 g(=95,39%) |
| 21 | 0,5/1,38/8 | Essigsäurepropyl= ester/ 0,15/55° | 58 | 31 — 58 | A=0,06 g(= 2,35%) B=2,54 g(=97,34%) C=0,008g(= 0,31%) | 1 — 58 | A=0,00 g(= 0,00%) B=0,01 g(= 0,71%) C=1,68 g(=99,29%) |
| 22 | 0,5/0,46/5 | Essigsäurebutyl= ester/ 0,2/55° | 75 | 40 — 75 | A=0,02 g(= 2,46%) B=1,10 g(=97,47%) C=0,0008g(= 0,07%) | 1 — 36 | A=0,00 g(= 0,00%) B=0,003g(= 1,16%) C=0,27 g(=98,84%) |
| 23 | 0,5/1,38/8 | Propionsäureäthyl= ester/ 0,18/55° | 86 | 45 — 86 | A=0,06 g(= 2,42%) B=2,41 g(=97,04%) C=0,01 g(= 0,54%) | 1 — 42 | A=0,00 g(= 0.00%) B=0,02 g(= 1,27%) C=1,60 g(=98,73%) |
| 24 | 0,5/1,4 /2 | Propionsäurepropyl= ester/ 0,25/55° | 70 | 37 — 70 | A=0,02 g(= 0,87%) B=2,52 g(=98,75%) C=0,01 g(= 0,38%) | 1 — 34 | A=0,00 g(= 0,00%) B=0,02 g(= 0,99%) C=1,69 g(=99,01%) |
| 25 | 1 /1,4 /2 | Buttersäureäthyl= ester/ 0,46/55° | 47 | 25 — 47 | A=0,04 g(= 0,84%) B=5,38 g(=98,79%) C=0,02 g(= 0,36%) | 1 — 23 | A=0,00 g(= 0,00%) B=0,03 g(= 0.82%) C=3,57 g(=99,18%) |

0 000 165

TABELLE II (Fortsetzung)

| Beispiel | Menge der wässr. Lösung [Liter] Fest- stoffgehalt [%] aus Beispiel 1 | org. Lösungsmittel/ Menge [Liter]/ Temperatur [°C] 2 | Extrak- tions stufen 3 | Solventphase | | Raffinatphase | |
|---|---|---|---|---|---|---|---|
| | | | | gewonnene Fraktionen | Gehalt 4 | gewonnene Fraktionen | Gehalt 5 |
| 26 | 0,5/0,46/5 | n-Butanol/ 0,075/55• | 86 | 41 — 86 | A=0.03 g(= 2,55%) B=0,99 g(=92,31%) C=0,05 g(= 5,14%) | 1 — 46 | A=0,00 g(= 0.00%) B=0,22 g(=48,24%) C=0,24 g(=51.76%) |
| 27 | 0,5/0,46/5 | n-Amylalkohol/ 0,08/55• | 86 | 40 — 86 | A=0,03 g(= 2,34%) B=0,96 g(=84.89%) C=0,14 g(=12,77%) | 1 — 49 | A=0,00 g(= 0,00%) B=0,50 g(=69,91%) C=0,22 g(=30,09%) |
| 28 | 0,5/1,38/8 | i-Amylalkohol/ 0,1/55• | 86 | 42 — 86 | A=0,06 g(= 2.32%) B=2,29 g(=90,25%) C=0,19 g(= 7,44%) | 1 — 45 | A=0,00 g(= 0,00%) B=0,28 g(=15,79%) C=1,52 g(=84,21%) |
| 29 | 0,5/1,38/8 | n-Hexanol/ 0.1/55• | 86 | 44 — 86 | A=0,06 g(= 2,41%) B=2,37 g(=96,03%) C=0,04 g(= 1,56%) | 1 — 43 | A=0,00 g(= 0,00%) B=0,06 g(= 3,61%) C=1,57 g(=96,39%) |
| 30 | 0,5/1,4 /2 | 2-Aethyl-butanol-(1)/ 0,14/7 55• | 86 | 42 — 86 | A=0,02 g(= 0.90%) B=2,28 g(=95,05%) C=0,10 g(= 4,05%) | 1 — 43 | A=0.00 g(= 0.00%) B=0,18 g(=10,27%) C=1,60 g(=89.73%) |

**0 000 165**

Beispiele 31—35
Dritte Extraktionsstufe der Gegenstromextraktion

Die in Beispiel 12 in den Fraktionen 1—15 der Raffinatphase gewonnene wäßrige Lösung wurde durch azeotropes Abdestillieren des Wassers auf einen Feststoffgehalt von 1,5 Gew.-% aufkonzentriert. Jeweils 0,1 l der aufkonzentrierten Lösung wurden in Element 1 einer 86 Stufen umfassenden Extraktionsapparatur vorlegt und bei 55°C mit verschiedenen organischen Lösungsmitteln einer Craig-Verteilung unterworfen.

In der nachstehenden Tabelle III sind in Spalte 1 die angewandten Lösungsmittel und Lösungsmittelmengen und in Spalten 2 und 3 der Gehalt der Solvent- bzw. Raffinatphase an 2-Hydroxybenzylalkohol (B), 4-Hydroxybenzylalkohol (D), 2,6-Dihydroxymethylphenol (E) und 2,4-Dihydroxymethylphenol und Polyhydroxymethylphenolen (F) sowie die zur Bestimmung des Gehaltes verwendeten Fraktionen aufgeführt. Die nicht aufgeführten Fraktionen wurden verworfen. Die Lösungsmittelmengen beziehen sich jeweils auf eine Extraktionsstufe.

Aus den angegebenen Daten geht hervor, daß mit Hilfe des erfindungsgemäßen Verfahrens ein, von Di- und Polyhydroxymethylphenolen praktisch freier 4-Hydroxybenzylalkohol erhalten wird.

TABELLE III

| Beispiel | org. Lösungsmittel/ Menge [Liter] 1 | Solventphase | | Raffinatphase | |
|---|---|---|---|---|---|
| | | gewonnene Fraktionen | Gehalt 2 | gewonnene Fraktionen | Gehalt 3 |
| 31 | Diisopropyläther 0,38 | 41 — 86 | B=0,002g(= 0,26%) D=0,64 g(=95,03%) E=0,03 g(= 4,72%) F=0,00 g(= 0,00%) | 1 — 46 | B=0,00g(= 0.00%) D=0,18g(=50,89%) E=0,11g(=29,97%) F=0,07g(=19,18%) |
| 32 | Pentanon-(3) 0,028 | 41 — 86 | B=0,002g(= 0,26%) D=0,64 g(=95,14%) E=0,03 g(= 4,61%) F=0,00 g(= 0,00%) | 1 — 46 | B=0,00g(= 0.00%) D=0,18g(=50,34%) E=0,11g(=30,40%) F=0,07g(=19,39%) |
| 33 | 4-Methyl-pentanon-(2) 0,039 | 42 — 86 | B=0,001g(= 0,22%) D=0,66 g(=97,50%) E=0,02 g(= 2,28%) F=0,00 g(= 0,00%) | 1 — 45 | B=0,00g(= 0,00%) D=0,09g(=34,28%) E=0.11g(=40.71%) F=0,07g(=25.00%) |
| 34 | n-Butanol 0,02 | 39 — 86 | B=0,001g(= 0,23%) D=0,63 g(=98,11%) E=0,01 g(= 1,66%) F=0,00 g(= 0,00%) | 1 — 41 | B=0,00g(= 0,00%) D=0,06g(=24.51%) E=0,12g(=46,23%) F=0,07g(=29,18%) |
| 35 | Octanol-(2) 0.048 | 38 — 86 | B=0,001g(= 0,21%) D=0,61 g(=88,80%) E=0,07 g(=10,99%) F=0,00 g(= 0,00%) | 1 — 49 | B=0,00g(= 0,00%) D=0,45g(=72,23%) E=0,10g(=16,18%) F=0,07g(=11,60%) |

**0 000 165**

Beispeil 36

(a) 0,5 l der wäßrigen Lösung des in Beispiel 1 (d) beschriebenen Reaktionsproduktes (Feststoffgehalt: 20 Gew.-%) wurden in einer 86 Stufen umfassenden Extraktionsapparatur einer Craig-Verteilung unterworfen. Die Lösung wurde in Element 1 vorgelegt und bei 55°C mit 4-Methyl-pentanon-(2) unter Anwendung eines Volumenverhältnisses $V_L/V_W$ von 0,39/1 extrahiert.

Die Analyse der Solvent- und Raffinatphasen ergab folgende Verteilung der Komponenten:

Element 1—45:
vereinigte Raffinatphasen:

0,00 g (= 0,00%) Phenol und Dihydroxydiphenylmethane

0,00 g (= 0,00%) 2-Hydroxybenzylalkohol

0,79 g (= 6,54%) 4-Hydroxybenzylalkohol

6,49 g (= 53,72%) 2,6-Dihydroxymethylphenol

4,80 g (= 39,73%) 2,4-Dihydroxymethylphenol und Polyhydroxymethylphenole

Elemente 42—86:
vereinigte Solventphasen:

19,09 g (= 33,80%) Phenol und Dihydroxydiphenylmethane

30,59 g (= 54,17%) 2-Hydroxybenzylalkohol

5,88 g (= 10,41%) 4-Hydroxybenzylalkohol

0,91 g (= 1,61%) 2,6-Dihydroxymethylphenol

(b) Die vereinigten Solventphasen der Elemente 42—86 des Beispiels 36 a) wurden auf 0,15 l eingeengt und bei 55°C mit Wasser unter Anwendung eines Volumenverhältnisses $V_L/V_W$ von 0,15/1 einer 59 Stufen umfassenden Craig-Verteilung unterworfen. Die Analyse der Solvent- und Raffinatphase ergab folgende Verteilung der Komponenten:

Elemente 1—28:
Vereinigte Raffinatphasen:

0,00 g (= 0,00%) Phenol und Dihydroxydiphenylmethane

0,04 g (= 1,08%) 2-Hydroxybenzylalkohol

3,66 g (= 98,92%) 4-Hydroxybenzylalkohol und 2,6-Dihydroxymethylphenol

Elemente 32—47:
Vereinigte Solventphasen:

0,26 g (= 1,48%) Phenol und Dihydroxydiphenylmethane

17,29 g (= 98,46%) 2-Hydroxybenzylalkohol

0,01 g (= 0,06%) 4-Hydroxybenzylalkohol

Elemente 48—59:
Vereinigte Solventphasen:

15,31 g (= 99,48%) Phenol und Dihydroxydiphenylmethane

0,08 g (= 0,52%) 2-Hydroxybenzylalkohol

0,00 g (= 0,00%) 4-Hydroxybenzylalkohol

16

# 0 000 165

## Beispiel 37

128 kg des in Beispiel 1 c) beschriebenen Sumpfproduktes wurden in der nachfolgend beschriebenen Gegenstromextraktion eingesetzt. Das Sumpfprodukt wurde durch Erwärmen auf 45°C (unter Stickstoff) verflüssigt und mit einer Geschwindigkeit von 2,668 kg je Stunde durch beheizte Leitungen zur ersten Extraktionskolonne gepumpt. Vor dem Eintritt in die Kolonne wurden in die Leitung je Stunde 1,232 kg auf 60°C erwärmtes destilliertes Wasser und 2,101 kg mit Wasser gesättigtes, auf 60°C erwärmtes Toluol gepumpt. Durch Einschaltung einer hinreichend langen Mischstrecke wurde für eine innige Durchmischung der 3 Flüssigkeitsströme gesorgt. Das so erhaltene Gemisch (Feed I) wies im zeitlichen Mittel folgende Zusammensetzung auf:

0,025 kg (= 0,42%) Kaliumacetat

1,321 kg (= 22,01%) Phenol

0,077 kg (= 1,28%) Dihydroxydiphenylmethane

0,815 kg (= 13,58%) 2-Hydroxybenzylalkohol

0,349 kg (= 5,80%) 4-Hydroxybenzylalkohol

0,057 kg (= 0,95%) 2,6-Dihydroxymethylphenol

0,023 kg (= 0,38%) 2,4-Dihydroxymethylphenol und Polyhydroxymethylphenole

1,235 kg (= 20,58%) Wasser

2,100 kg (= 34,99%) Toluol

---

6,002 kg (= 100 %) Feed I

Das Gemisch (Feed I) wurde in die Mitte einer Glas-Siebboden-Kolonne (Kolonne 1; Gesamtlänge: 5,50 m; innerer Durchmesser: 50 mm; Bodenabstände: 50 mm; Anzahl der praktischen Böden: 90) eingeleitet Die Kolonne wurde durch eine Mantelheizung auf 60°C und mittels Stickstoff auf einem Überdruck von 0,4 bar gehalten.

Oberhalb des unteren Abscheiders wurden je Stunde 2,154 kg mit Wasser gesättigtes, auf 60°C erwärmtes Toluol und unterhalb des oberen Abscheiders je Stunde 3,893 kg auf 60°C erwärmtes destilliertes Wasser eindosiert. Die für den Stoffaustausch erforderlichen kleinen Flüssigkeitströpfchen der Extraktionsmittel wurden durch Pulsation (Hub: 2—3 mm; Frequenz: 100 Hübe pro Minute) erzeugt. Die Lage der Trennschicht zwischen beiden Phasen wurde durch eine Elektrode, die auf die unterschiedlichen Dielektrizitätskonstanten der beiden Phasen anspricht, konstant gehalten.

Aus dem oberen Abscheider liefen im Durchschnitt je Stunde 5,766 kg Solventphase und aus dem unteren Abscheider im Durchschnitt je Stunde 6,284 kg Raffinatphase ab. Solvent- und Raffinatphase hatten folgende Zusammensetzung:

17

| Solventphase | | Raffinatphase | | |
|---|---|---|---|---|
| — | — | 0,025kg | (= 0,40%) | Kaliumacetat |
| 1,295kg | (=22,46%) | 0,026kg | (= 0,42%) | Phenol |
| 0,077kg | (= 1,33%) | — | — | Dihydroxydiphenyl-methane |
| 0,058kg | (= 1,00%) | 0,757kg | (=12,05%) | 2-Hydroxy-benzylalkohol |
| — | — | 0,349kg | (≈ 5,56%) | 4-Hydroxy-benzylalkohol |
| — | — | 0,057kg | (= 0,91%) | 2,6-Dihydroxymethyl-phenol |
| — | — | 0,023kg | (= 0,37%) | 2,4-Dihydroxymethyl-phenol + Polyhydroxymethyl-phenole |
| 0,115kg | (= 2,00%) | 5,015kg | (=79,80%) | Wasser |
| 4.221kg | (=73,21%) | 0,032kg | (= 0,50%) | Toluol |
| 5,766kg | (=100%) | 6,284kg | (=100%) | Summe |

Die Solventphase wurde kontinuierlich im Fallfilm- und Dünnschichtverdampfer im Vakuum vom Lösungsmittel befreit.

Die je Stunde anfallenden 6,284 kg Raffinatphase wurden zunächst durch einen Toluol-Stripper geleitet, um das gelöste Toluol azeotrop zu entfernen. Die bei der Destillation je Stunde anfallenden 6,250 kg toluolfreie wäßrige Lösung (Feed II) wurden in die Mitte einer zweiten Siebbodenkolonne (Kolonne 2; Gesamtlänge: 9 m; innerer Durchmesser: 72.5 mm; Bodenabstände: 100 mm) eindosiert. Kolonne 2 wurde durch eine Mantelheizung auf 55°C und mittels Stickstoff auf einem Überdruck von 0,65 bar gehalten.

In den unteren Zulaufstutzen der Kolonne wurden je Stunde 5,447 kg mit Wasser gesättigter, auf 55°C erwärmter Diisopropyläther, in den oberen Zulaufstutzen je Stunde 1,179 kg auf 55°C erwärmtes Wasser eindosiert. Die für den Stoffaustausch erforderlichen kleinen Flüssigkeitströpfchen der Extraktionsmittel wurden durch Pulsation (Hub: 1,5 — 2 mm; Frequenz: 103 Hübe je Minute) erzeugt. Die Lage der Trennschicht wurde wie in Kolonne 1 durch eine in der Soll-Lage der Trennschicht angebrachte Elektrode konstant gehalten.

Aus dem oberen Abscheider liefen durchschnittlich je Stunde 6,175 kg Solventphase, aus dem unteren Abscheider je Stunde 6,7 kg Raffinatphase ab. Solvent- und Raffinatphase hatten im zeitlichen Mittel folgende Zusammensetzung:

| Solventphase | | Raffinatphase | | |
|---|---|---|---|---|
| — | — | 0,025kg | (= 0,37%) | Kaliumacetat |
| 0,026kg | (= 0,42%) | — | — | Phenol |
| — | — | — | — | Dihydroxydiphenyl-methane |
| 0,757kg | (=12,26%) | — | — | 2-Hydroxy-benzylalkohol |
| 0,012kg | (= 0,19%) | 0,337kg | (= 5,03%) | 4-Hydroxy-benzylalkohol |
| — | — | 0,057kg | (= 0,85%) | 2,6-Dihydroxymethyl-phenol |
| — | — | 0,023kg | (= 0,34%) | 2,4-Dihydroxymethyl-phenol + Polyhydroxymethyl-phenole |
| 0,062kg | (= 1,00%) | 6,191kg | (=92,40%) | Wasser |
| 5,318kg | (=86,12%) | 0,067kg | (= 1,00%) | Diisopropyläther |
| 6,175kg | (=100%) | 6,700kg | (=100%) | Summe |

Die Solventphase der Kolonne 2 wurde kontinuierlich im Fallfilm- und Dünnschichtverdampfer vom Lösungsmittel befreit.

Der in den je Stunde anfallenden 6,700 kg Raffinatphase gelöste Diisopropyläther wurde in einem Stripper azeotrop abdestilliert. Die je Stunde anfallenden 6,632 kg diisopropylätherfreie wäßrige Lösung (Feed III) wurden in die Mitte einer dritten Siebbodenkolonne (Kolonne 3; Gesamtlänge: 9 m; innerer Durchmesser: 72,5 mm; Bodenabstände: 100 mm) eindosiert. Kolonne 3 wurde durch eine Mantelheizung auf 55°C und durch Stickstoff auf einem Überdruck von 0,5 bar gehalten. In den unteren Zulaufstutzen von Kolonne 3 wurden je Stunde 2,704 kg auf 55°C erwärmtes, mit Wasser gesättigtes 4-Methyl-pentanon-(2), in den oberen Zulaufstutzen je Stunde 2,520 kg auf 55°C erwärmtes destilliertes Wasser dosiert. Die für den Stoffaustausch erforderlichen kleinen Flüssigkeitströpfchen der Extraktionsmittel wurden durch Pulsation (Hub 1,5 bis 2 mm; Frequenz: 110 bis 120 Hübe je Minute) erzeugt. Die Lage der Trennschicht wurde wie in den Kolonnen 1 und 2 durch eine in der Soll-Lage der Trennschicht angebrachte Elektrode konstant gehalten.

Aus dem oberen Abscheider liefen durchschnittlich je Stunde 2,871 kg Solventphase, aus dem unteren Abscheider je Stunde 8,985 kg Raffinatphase ab. Solvent- und Raffinatphase wiesen im zeitlichen Mittel folgende Zusammensetzung auf:

| Solventphase | | Raffinatphase | | |
|---|---|---|---|---|
| — | — | 0,025kg | (= 0,28%) | Kaliumacetat |
| — | — | — | — | Phenol |
| — | — | — | — | Dihydroxydiphenyl-methane |
| — | — | — | — | 2-Hydroxy-benzylalkohol |
| 0,327kg | (=11,39%) | 0,010kg | (= 0,11%) | 4-Hydroxy-benzylalkohol |
| 0,018kg | (= 0,63%) | 0,039kg | (= 0,43%) | 2,6-Dihydroxymethyl-phenol |
| — | — | 0,023kg/h | (= 0,25%) | 2,4-Dihydroxymethyl-phenol + Polyhydroxymethyl-phenole |
| 0,051kg | (≑ 1,78%) | 8,711kg | (=96,95%) | Wasser |
| — | — | — | — | Diisopropyläther |
| — | — | — | — | Toluol |
| 2,475kg | (=86,21%) | 0,177kg | (= 1,97%) | 4-Methyl-pentanon-(2) |
| 2,871kg | (=100%) | 8,985kg | (=100%) | Summe |

Die Solventphase der Kolonne 3 wurde kontinuierlich im Fall- film- und Dünnschichtverdampfer vom Lösungsmittel befreit, die Raffinatphase wurde verworfen.

Die vorstehend beschriebene 3-stufige Gegenstrom-Extraktion wurde insgesamt 48 Stunden durchgeführt. Nach etwa 24 Stunden war die gesamte Anlage im Gleichgewicht. Die in der nachstehenden Tabelle IV angegebene Feststoffbilanz bezieht sich auf die letzten 24 Stunden.

TABELLE IV

| | Durchsatz in 24 h | In der Sol-ventphase von Kolonne 1 ent-haltene Fest-stoffe | In der Sol-ventphase von Kolonne 2 ent-haltene Fest-stoffe | In der Sol-ventphase von Kolonne 3 ent-haltene Fest-stoffe | In der Raffi-natphase von Kolonne 3 ent-haltene Fest-stoffe |
|---|---|---|---|---|---|
| Phenol | 31,704 kg | 31,080 kg (= 90,56%) | 0,624 kg (= 3,27%) | — | — |
| Dihydroxydiphenylmethan | 1,848 kg | 1,848 kg (= 5,382%) | — | — | — |
| 2-Hydroxybenzylalkohol | 19,560 kg | 1,392 kg (= 4,06%) | 18,168 kg (= 95,22%) | — | — |
| 4-Hydroxybenzylalkohol | 8,376 kg | — | 0,288 kg (= 1,51 %) | 7,848 kg (= 94,78%) | 0,240 kg (= 13,89%) |
| 2,6-Dihydroxymethylphenol | 1,368 kg | — | — | 0,432 kg (= 5,22%) | 0,936 kg (= 54,17%) |
| 2,4-Dihydroxymethylphenol und Polyhydroxymethylphenol | 0,552 kg | — | — | — | 0,552 kg (= 31,94%) |
| | 63,408 kg | 34,320 kgg (= 100%) | 19,080 kg (= 100%) | 8,280 kg (= 100%) | 1,728 kg (= 100%) |

0 000 165

**0 000 165**

**Patentansprüche**

1. Verfahren zur Herstellung von reinem 2-Hydroxybenzylalkohol und reinem 4-Hydroxybenzylalkohol oder einem Gemisch beider Verbindungen durch Umsetzung von Phenol mit Formaldehyd in Gegenwart eines basischen Katalysators und Extraktion der wäßrigen Lösung des erhaltenen Reaktionsgemisches mit einem organischen Lösungsmittel, gegebenenfalls nach Entfernen eines Teils des nicht umgesetzten Phenols, dadurch gekennzeichnet, daß man das Dihydroxydiphenylmethane, Phenol, 2-Hydroxybenzylalkohol, 4-Hydroxybenzylalkohol, Dihydroxymethylphenole und Polyhydroxymethylphenole enthaltende Reaktionsgemisch einer mindestens 2-stufigen Gegenstromextraktion in einem Lösungsmittelsystem Wasser/mit Wasser nicht oder begrenzt mischbares organisches Lösungsmittel unterwirft, wobei man die Gegenstromextraktion in der Weise ausführt, daß man entweder

(a) in der ersten Extraktionsstufe als organische mit Wasser nicht bzw. begrenzt mischbare Lösungsmittel Alkylaromaten mit 7 oder 8 C-Atomen, cyclische Kohlenwasserstoffe mit 6 — 8 C-Atomen oder aliphatische 6—8 C-Atome aufweisende Äther einsetzt und ein Volumenverhältnis $V_L/V_W$ anwendet, so daß sich eine Phenol und Dihydroxydiphenylmethane enthaltende organische und eine die übrigen Verbindungen enthaltende wäßrige Phase bildet, die organische Phase abtrennt und die wäßrige Phase in der zweiten Extraktionsstufe einer Gegenstromverteilung mit aliphatischen 6 — 8 C-Atome aufweisenden Äthern oder aliphatischen 5—6 C-Atome aufweisenden Ketonen unterwirft, so daß sich eine organische Phase, die entweder 2-Hydroxybenzylalkohol oder 2- und 4-Hydroxybenzylalkohol enthält und eine wäßrige Phase, die die restlichen Verbindungen enthält, bildet, die organische Phase abtrennt und, falls in der zweiten Extraktionsstufe 2-Hydroxybenzylalkohol abgetrennt wurde, die wäßrige Phase in einer dritten Extraktionsstufe einer Gegenstromverteilung mit aliphatischen, 5 — 6 C-Atome aufweisenden Ketonen oder aliphatischen 4 — 8 C-Atome aufweisenden Alkoholen unterwirft, so daß sich eine den 4-Hydroxybenzylalkohol enthaltende organische Phase und eine die Polyhydroxymethylphenole enthaltende wäßrige Phase bildet, oder

(b) in der ersten Extraktionsstufe als organische mit Wasser nicht bzw. begrenzt mischbare Lösungsmittel aliphatische 5—6 C-Atome aufweisende Ketone einsetzt und ein Volumenverhältnis $V_L/V_W$ anwendet, so daß sich eine die Polyhydroxymethylphenole enthaltende wäßrige Phase und eine die übrigen Verbindungen enthaltende organische Phase bildet, die wäßrige Phase abtrennt und die organische Phase in einer zweiten Extraktionsstufe einer Gegenstromverteilung mit Wasser unterwirft, so daß sich eine wäßrige Phase, die entweder 4-Hydroxybenzylalkohol oder 4- und 2-Hydroxybenzylalkohol enthält, und einde organische Phase bildet, die die übrigen Verbindungen enthält, die wäßrige Phase abtrennt und, falls in der zweiten Extraktionsstufe nur 4-Hydroxybenzylalkohol extrahiert wurde, die organische Phase in einer dritten Extraktionsstufe einer Gegenstromverteilung mit Wasser unterwirft, so daß sich eine den 2-Hydroxybenzylalkohol enthaltende organische und eine die restlichen Verbindungen enthaltende wäßrige Phase bildet.

2. Verfahren gemäß Anspruch 1, dadurch gekennzeichnet, daß man als organisches Lösungsmittel in der Verfahrensalternative a in der ersten Extraktionsstufe Toluol, in der zweiten Extraktionsstufe Diisopropyläther und in der dritten Extraktionsstufe Methyl-isobutylketon verwendet.

3. Verfahren gemäß Anspruch 1 und 2, dadurch gekennzeichnet, daß in dem für die Gegenstromextraktion angewendeten System Wasser/mit Wasser nicht oder begrenzt mischbares organisches Lösungsmittel das Volumenverhältnis $V_L/V_W$ 0,05 bis 20 beträgt.

4. Verfahren gemäß Anspruch 1 bis 3, dadurch gekennzeichnet, daß man die Gegenstromextraktion bei Temperaturen von 20 bis 70°C ausführt.

5. Verfahren gemäß Anspruch 1 bis 4, dadurch gekennzeichnet, daß man Phenol mit Paraformaldehyd im Molverhältnis 5 bis 15:1 umsetzt.

6. Verfahren gemäß Anspruch 1 bis 4, dadurch gekennzeichnet, daß man Phenol mit Paraformaldehyd im Molverhältnis 8 bis 12:1 umsetzt.

**Revendications**

1. Procédé de production d'alcool 2-hydroxybenzylique pur et d'alcool 4-hydroxybenzylique pur, ou d'un mélange des deux composés par réaction de phénol avec le formaldéhyde en présence d'un catalyseur basique et extraction de la solution aqueuse du mélange réactionnel obtenu avec un solvant organique, éventuellement avec élimination d'une partie du phénol n'ayant pas réagi, procédé qui est caractérisé en ce qu'on soumet le mélange réactionnel contenant des dihydroxydiphénylméthanes, du phénol, de l'alcool 2-hydroxybenzylique, de l'alcool 4-hydroxybenzylique, des dihydroxyméthylphénols et des polyhydroxyméthylphénols à une extraction à contre-courant en au moins deux étapes dans un mélange de solvants formé d'eau et d'un solvant organique non miscible ou partiellement miscible à l'eau, et on conduit l'extraction à contre-courant de façon telle que:

(a) on utilise dans la première étape d'extraction comme solvants organiques non miscibles à

## 0 000 165

l'eau ou partiellement miscibles à l'eau, des hydrocarbures alkylaromatiques ayant 7 ou 8 atomes de carbone, des hydrocarbures cycliques ayant 6 à 8 atomes de carbone ou des éthers aliphatiques comprenant 6 à 8 atomes de carbone et on utilise un rapport en volume $V_{solvant}/V_{eau}$ de manière qu'il se forme une phase organique contenant du phénol et des dihydroxydiphénylméthanes et une phase aqueuse contenant les autres composés, on sépare la phase organique et on soumet la phase aqueuse dans la seconde étape d'extraction à une répartition à contre-courant avec des éthers aliphatiques contenant 6 à 8 atomes de carbone ou des cétones aliphatiques comprenant 5 ou 6 atomes de carbone, de manière qu'il se forme une phase organique qui contient ou bien l'alcool 2-hydroxybenzylique ou bien les alcools 2- et 4-hydroxybenzyliques et une phase aqueuse qui contient les composés restants, on sépare la phase organique et, au cas où l'alcool 2-hydroxybenzylique a été séparé dans la seconde étape d'extraction, on soumet la phase aqueuse dans une troisième étape d'extraction à une répartition à contre-courant avec des cétones aliphatiques ayant 5 ou 6 atomes de carbone ou des alcools aliphatiques ayant 4 à 8 atomes de carbone, de manière qu'il se forme une phase organique contenant l'alcool 4-hydroxybenzylique et une phase aqueuse contenant les polyhydroxyméthylphénols, ou bien

(b) on utilise comme solvants organiques non miscibles à l'eau ou partiellement miscibles à l'eau dans la première étape d'extraction, des cétones aliphatiques ayant 5 ou 6 atomes de carbone et un rapport en volume $V_{solvant}/V_{eau}$ choisi de manière qu'il se forme une phase aqueuse contenant les polyhydroxyméthylphénols et une phase organique contenant les autres composés, on sépare la phase aqueuse et on soumet la phase organique dans une seconde étape d'extraction à une répartition à contre-courant avec de l'eau, de manière qu'il se forme une phase aqueuse qui contient ou bien l'alcool 4-hydroxybenzylique ou bien les alcools 4- et 2-hydroxybenzyliques, et une phase organique qui contient les autres composés, on sépare la phase aqueuse et, au cas où l'alcool 4-hydroxybenzylique a seul été extrait dans la seconde étape d'extraction, on soumet la phase organique dans une troisième étape d'extraction à une répartition à contre-courant avec de l'eau, de manière qu'il se forme une phase organique contentant l'alcool 2-hydroxybenzylique et une phase aqueuse contenant les composés restants.

2. Procédé suivant la revendication 1, caractérisé en ce qu'on utilise comme solvant organique dans la variante (a), du toluène dans la première étape d'extraction, de l'éther de diisopropyle dans la deuxième étape d'extraction et de la méthylisobutylcétone dans la troisième étape d'extraction.

3. Procédé suivant les revendications 1 et 2, caractérisé en ce que le rapport en volume $V_s/V_E$ a une valeur de 0,05 à 20 dans le système eau/solvant organique non miscible ou partiellement miscible à l'eau utilisé pour l'extraction à contre-courant.

4. Procédé suivant les revendications 1 à 4, caractérisé en ce que l'extraction à contre-courant est conduite à des températures de 20 à 70°C.

5. Procédé suivant les revendications 1 à 4, caractérisé en ce qu'on fait réagir du phénol avec du paraformaldéhyde dans le rapport molaire de 5 à 15:1.

6. Procédé suivant les revendications 1 à 4, caractérisé en ce qu'on fait réagir du phénol avec du paraformaldehyde dans un rapport molaire de 8 à 12:1.

**Claims**

1. Process for the preparation of pure 2-hydroxybenzyl alcohol and pure 4-hydroxybenzyl alcohol or a mixture of both compounds by reacting phenol with formaldehyde in the presence of a basic catalyst and extracting the aqueous solution of the resulting reaction mixtures with an organic solvent, optionally after removing some of the unreacted phenol, characterised in that the reaction mixture containing dihydroxydiphenylmethanes, phenol, 2-hydroxybenzyl alcohol, 4-hydroxybenzyl alcohol, dihydroxymethylphenols and polyhydroxymethylphenols is subjected to an at least 2-stage countercurrent extraction in a solvent system of water/organic solvent which is water-immiscible or water-miscible only to a limited extent, wherein the countercurrent extraction is carried out, in such a way that either

(a) in the first extraction stage alkylaromatic compounds with 7 or 8 C atoms, cyclic hydrocarbons with 6 to 8 C atoms or aliphatic ethers containing 6 to 8 C atoms are used as the organic solvents which are water-immiscible or water-miscible to a limited extent and a volume ratio $V_s/V_w$ is applied so that an organic phase containing phenol and dihydroxydiphenylmethanes and an aqueous phase containing the remaining compounds are formed, the organic phase is separated off and the aqueous phase is subjected in the second extraction phase to countercurrent distribution with aliphatic ethers containing 6 to 8 C atoms or aliphatic ketones containing 5 to 6 C atoms, so that an organic phase, which contains either 2-hydroxybenzyl alcohol or 2- and 4-hydroxybenzyl alcohol, and an aqueous phase, which contains the remaining compounds, are formed, the organic phase is separated off and, if 2-hydroxybenzyl alcohol was separated off in the second extraction stage, the aqueous phase is subjected, in a third extraction stage, to countercurrent distribution with aliphatic ketones containing 5 to 6 C atoms or aliphatic alcohols containing 4 to 8 C atoms, so that an organic phase containing the

22

# 0 000 165

4-hydroxybenzyl alcohol and an aqueous phase containing the polyhydroxymethylphenols are formed, or

(b) in the first extraction stage aliphatic ketones containing 5 to 6 C atoms are used as the organic solvents which are water immiscible or water-miscible to a limited extent and a volume ratio $V_s/V_w$ is applied so that an aqueous phase containing the polyhydroxymethylphenols and an organic phase containing the remaining compounds are formed, the aqueous phase is separated off and the organic phase is subjected in a second extraction stage to countercurrent distribution with water, so that an aqueous phase, which contains either 4-hydroxybenzyl alcohol or 4- and 2-hydroxybenzyl alcohol, and an organic phase, which contains the remaining compounds, are formed, the aqueous phase is separated off and, if only 4-hydroxybenzyl alcohol was extracted in the second extraction stage, the organic phase is subjected, in a third extraction stage, to countercurrent distribution with water, so that an organic phase containing the 2-hydroxybenzyl alcohol and an aqueous phase containing the remaining compounds are formed.

2. Process according to Claim 1, characterised in that the organic solvent used in the process alternative (a) is toluene in the first extraction stage, diisopropyl ether in the second extraction stage and methyl isobutyl ketone in the third extraction stage.

3. Process according to Claim 1 and 2, characterised in that the volume ratio $V_s/V_w$ in the system of water/organic solvent which is water-immiscible or water-miscible to a limited extent used for the countercurrent extraction is 0.05 to 20.

4. Process according to Claim 1 to 4 characterised in that the countercurrent extraction is carried out at temperatures from 20 to 70°C.

5. Process according to Claim 1 to 4, characterised in that phenol is reacted with paraformaldehyde in the molar ratio of 5—15:1.

6. Process according to Claim 1 to 4, characterised in that phenol is reacted with paraformaldehyde in the molar ratio 8—12:1.

23